# EUROPÄISCHE PATENTANMELDUNG

(11) **EP 0 848 913 A2**
(43) Veröffentlichungstag der Anmeldung: **24.06.1998**
(21) Anmeldenummer: 97122115.5
(22) Anmeldetag: 16.12.1997
(51) Int. Cl.: A23L 1/275, A23C 9/152, A61K 9/107, A23L 2/58, A23D 7/00

(54) **Verwendung von Carotinoid-Solubilisaten zum Färben von Lebensmitteln und pharmazeutischen Zubereitungen**

(30) Priorität: 20.12.1996 DE 19653410
(71) Anmelder: BASF AKTIENGESELLSCHAFT, 67056 Ludwigshafen (DE)
(72) Erfinder: Runge, Frank, Dr., 67133 Maxdorf (DE); Zwissler, Georg Konrad, Dr., 67098 Bad Dürkheim (DE); End, Lutz, Dr., 68199 Mannheim (DE); Schweikert, Loni, Dr., 67122 Altrip (DE); Horn, Dieter, Dr., 69120 Heidelberg (DE)

(57) **Zusammenfassung**

Verwendung von Carotinoid-Solubilisaten zum Färben von Lebensmitteln und pharmazeutischen Zubereitungen, dadurch gekennzeichnet, daß man Carotinoid-Solubilisate einsetzt, die hergestellt sind, indem man eine Suspension, enthaltend 1 bis 40 Gew.-% eines oder mehrerer Carotinoide, 20 bis 90 Gew.-% eines oder mehrerer nichtionogener Emulgatoren und 0 bis 50 Gew.-% weiterer Zusatzstoffe, kurzzeitig auf Temperaturen von 120 bis 200°C erhitzt, und die homogene Lösung mit solchen Mengen Wasser oder einer wäßrigen Lösung, enthaltend hydrophile Antioxidantien sowie gegebenenfalls weitere grenzflächenaktive Zusatzstoffe, bei 10 bis 95°C turbulent vermischt, daß ein Solubilisat mit 0,5 bis 10 Gew.-% an Carotinoiden entsteht.

## Beschreibung

Die Erfindung betrifft die Verwendung von Carotinoid-Solubilisaten mit einem Carotinoid Gehalt von 0,5 bis 10 Gew.-% zum Färben von Lebensmitteln und pharmazeutischen Zubereitungen.

Carotinoide bilden eine Gruppe von Farbpigmenten mit gelber bis roter Farbtonnuance, die in der Natur weitverbreitet vorkommen und vielen Nahrungsmitteln eine charakteristische Färbung verleihen. Als wichtigste Vertreter dieser Stoffklasse seien β-Carotin, β-Apo-8'-carotinal, Canthaxanthin, Asthaxanthin, Lycopin und Citranaxanthin genannt. Sowohl für die Lebensmittelindustrie als auch für die pharmazeutische Technologie stellen diese synthetisch herstellbaren Substanzen z.B. als Ersatz für künstliche Farbstoffe wichtige Farbkörper dar und sind zum Teil wegen ihrer Pro-Vitamin-A-Aktivität von Interesse.

Alle Carotinoide sind in Wasser unlöslich, während in Fetten und Ölen eine jedoch nur geringe Löslichkeit gefunden wird. Diese begrenzte Löslichkeit sowie die hohe Oxidationsempfindlichkeit stehen einer direkten Anwendung der relativ grobkörnigen bei der Synthese erhaltenen Produkte in der Einfärbung von Lebensmitteln entgegen, da die Substanzen in grobkristalliner Form nur schlechte Färbungsergebnisse liefern.

Zur Verbesserung der Farbausbeuten sind verschiedene Verfahren beschrieben worden, die alle das Ziel haben, die Kristallitgröße der Wirkstoffe zu verkleinern und auf einen Teilchengrößenbereich von kleiner 10 µm zu bringen. Neben der Vermahlung von Carotinoiden, gemäß WO 91/06292 bzw. WO 94/19411, zählen dazu beispielsweise die bekannten Emulgier- und Mikronisierverfahren, u.a. beschrieben in DE-A-12 11 911, EP-A-0 410 236 sowie in EP-B-0 065 193.

Für spezielle Anwendungsgebiete von Carotinoiden, beispielsweise für die Färbung von Getränken (u.a. Soft-drinks) ist gewünscht, daß die Carotinoid-Formulierung in flüssiger Form vorliegt, und daß die Redispergierung dieser Flüssigformulierung in wässrigen Systemen zu klar gefärbten Lösungen führt. Um diesen Effekt zu erzielen, sind entsprechend kleine Wirkstoffpartikel (<100 nm) erforderlich.

In der EP-A-0 551 638 werden Emulsionen von β-Carotin beschrieben, die mit Ascorbylpalmitat als Emulgator stabilisiert sind, deren Teilchengröße jedoch zwischen 200 und 300 nm liegt.

Weiterhin sind in WO 94/06310 Carotinoid-Solubilisate zur Getränkefärbung beschrieben, deren Wirkstoffgehalt allerdings nur bei maximal 1,0 Gew.-% liegt.

Es war daher die Aufgabe, stabile Carotinoid-Solubilisate zum Färben von Lebensmitteln und pharmazeutischen Zubereitungen vorzuschlagen, deren Teilchengröße im Bereich von 10 bis 200 nm liegt und deren Wirkstoffkonzentration größer 0,5 Gew.-% beträgt.

Die Aufgabe wurde erfindungsgemäß gelöst durch die Verwendung von Carotinoid-Solubilisaten zum Färben von Lebensmitteln und pharmazeutischen Zubereitungen, dadurch gekennzeichnet, daß man Carotinoid-Solubilisate einsetzt, die hergestellt sind, indem man eine Suspension, enthaltend 1 bis 40 Gew.-% eines oder mehrerer Carotinoide, 20 bis 90 Gew.-% eines oder mehrerer nichtionogener Emulgatoren und 0 bis 50 Gew.-% weiterer Zusatzstoffe, kurzzeitig auf Temperaturen von 120 bis 200°C erhitzt, und die homogene Lösung mit solchen Mengen Wasser oder einer wäßrigen Lösung, enthaltend hydrophile Antioxidantien sowie gegebenenfalls weitere grenzflächenaktive Zusatzstoffe, bei 10 bis 95°C turbulent vermischt, daß ein Solubilisat mit 0,5 bis 10 Gew.-% an Carotinoiden entsteht. Das Solubilisat kann gegebenenfalls auf eine gewünschte Endkonzentration weiter verdünnt werden.

Die erfindungsgemäße Verwendung der Carotinoid-haltigen Solubilisate in der Lebensmittelfärbung ermöglicht es, ein breites Farbspektrum abdecken zu können, da auf Basis konzentrierter Wirkstoff-Formulierungen der gewünschte Farbeffekt über die Menge der entsprechenden Carotinoid-haltigen Formulierung eingestellt werden kann.

Die Herstellung der für die erfindungsgemäße Verwendung als Lebensmittelfarbstoff eingesetzten Solubilisate kann sowohl diskontinuierlich, gemäß EP-A-0 055 817 als auch insbesondere kontinuierlich nach dem in EP-A-0 479 066 beschriebenen Verfahren erfolgen. Dabei wird eine bevorzugt auf 20 bis 80°C, vorzugsweise 50 bis 70°C vorgewärmte Suspension von 1 bis 40 Gew.-%, bevorzugt 10 bis 30 Gew.-% eines oder mehrerer Carotinoide in 20 bis 90 Gew.-% eines oder mehrerer nichtionogener Emulgatoren, gegebenenfalls unter Zugabe von 0 bis 50 Gew.-% weiterer Zusatzstoffe, durch eine in einem Wärmeträgeröl befindliche Heizschlange gepumpt, wobei die Temperatur in dem Solubilisierungsgemisch 120 bis 200°C und die Verweilzeit 10 bis 300 Sekunden beträgt und die homogene Lösung in einer Mischkammer, ggf. unter einem auf 10 bis 50 bar erhöhten Druck mit solchen Mengen Wasser oder einer wäßrigen Lösung, enthaltend hydrophile Antioxidantien sowie gegebenenfalls weitere grenzflächenaktive Zusatzstoffe, von 10 bis 95°C turbulent vermischt, daß eine schnelle Abkühlung der homogenen Lösung auf unter 95°C erfolgt und ein Solubilisat mit 0,5 bis 10 Gew.-%, vorzugsweise 1,5 bis 6 Gew.-% Carotinoid entsteht.

Bezüglich genauerer Verfahrensbeschreibungen wird hiermit auf die beiden o.g. europäischen Patentschriften und die dort beschriebenen Bedingungen ausdrücklich Bezug genommen.

Die Carotinoide, die bei der Durchführung der Erfindung eingesetzt werden können, sind die bekannten, zugänglichen, natürlichen oder synthetischen Vertreter dieser Klasse von Verbindungen, die als farbgebende Mittel brauchbar sind, z.B. β-Carotin, Lycopin, Bixin, Zeaxanthin, Cryptoxanthin, Citranaxanthin, Lutein, Canthaxanthin, Astaxanthin, β-Apo-4'-carotinal, β-Apo-8'-carotinal, β-Apo-12'-carotinal, β-Apo-8'-carotinsäure sowie Ester von hydroxy- und carboxyhaltigen Vertretern dieser Gruppe, z.B. die niederen Alkylester und vorzugsweise die Methyl- und Ethylester. Besonders bevorzugt werden die bisher technisch gut zugänglichen Vertreter wie β-Carotin, Canthaxanthin, Astaxanthin, Lycopin, β-Apo-8'-carotinal und β-Apo-8'-carotinsäureester verwendet.

Die Carotinoide können dabei sowohl in reiner Form als auch in Form einer öligen Dispersion im Emulgator suspendiert werden, wobei das Dispersionsmittel sowohl mineralischen, pflanzlichen als auch tierischen Ursprungs sein kann. Typische Vertreter sind eßbare Öle, insbesondere Sesamöl, Maiskeimöl, Baumwollsaatöl, Sojabohnenöl oder Erdnußöl.

Als Emulgatoren kommen an sich bekannte nicht-ionogene Emulgatoren mit einem HLB-Wert (vgl. H.P. Fiedler, Lexikon der Pharmazie, Kosmetik und angrenzende Gebiete, 1996, Seiten 753-756) von 12 bis 16 in Betracht, insbesondere ethoxylierte Triglyceride von Fettsäuren mit 12 bis 18 Kohlenstoffatomen, die 20 bis 60 Oxyethyleneinheiten enthalten oder ethoxylierte Sorbitanfettsäureester mit etwa 20 Oxyethyleneinheiten oder ethoxylierte Monohydroxyfettsäuren mit 14 bis 17 Oxyethyleneinheiten wie sie in der DE-A-29 11 241 beschrieben sind. Derartige Emulgatoren heißen auch Solubilisatoren, weil sie sich in Wasser lösen und dadurch als Lösungsvermittler für lipophile Substanzen wirken, indem diese in micellarer Lösung gehalten werden. Micellare Lösungen zeichnen sich durch Transparenz und Klarheit aus. Sie können charakterisiert werden durch die Angabe der Teilchengröße der Micellen, bestimmt durch quasielastische Lichtstreuung.

Entsprechende Durchmesser liegen zwischen 10 und 200 nm, je nach verwendetem Solubilisator und Wirkstoffgehalt.

Als besonders geeignete nicht-ionogene Emulgatoren seien beispielsweise genannt: Glycerinpolyoxyethylenglykolricinoleat, Glycerinpolyoxyethylenglykoloxystearat, Polyoxyethylen(20)-sorbitanmonooleat, Polyoxyethylen(20)sorbitanmonostearat, Polyoxyethylen(20)sorbitanmonopalmitat und Monohydroxystearinsäure mit 15 Oxethyleneinheiten, die in einer Konzentration von 100 bis 1000 Gew.-%, bevorzugt 300 bis 800 Gew.-%, bezogen auf das eingesetzte Carotinoid, verwendet werden.

Zur Verbesserung der Antischaumeigenschaften der Solubilisate können zusätzlich zu den bereits beschriebenen Solubilsatoren weitere grenzflächenaktive Substanzen, bevorzugt in einer Konzentration von 0 bis 5 Gew.-%, bezogen auf die Gesamtmenge des Solubilisates, eingesetzt werden. Neben Fettalkoholen, Ölen, Polymeren wie z.B. Polysiloxanen und Phospholipiden wie z.B. Lecithin sind es bevorzugt Emulgatoren mit einem HLB-Wert kleiner 5, die als Entschäumer wirken. Diese Antischaummittel können bei der Solubilisatherstellung je nach Löslichkeit entweder zur Solubilisator/Carotinoid-Mischung oder zur wäßrigen Phase gegeben werden. Es ist aber auch möglich, die Entschäumer erst nach der Solubilisat-Herstellung separat zuzusetzen.

Zum Schutz der Carotinoide vor oxidativem Abbau und damit zur Erhöhung der Lichtstabilität der verwendeten Solubilisate können zusätzlich Antioxidantien mit verwendet werden. Als übliche Antioxidantien, die gegebenenfalls im Solubilisat einzeln oder als Gemisch in Mengen von 0 bis 500 Gew.-% im Falle von wasserlöslichen Antioxidantien und 0 bis 200 Gew.-% im Falle von öllöslichen Antioxidantien, bezogen auf eingesetztes Carotinoid, enthalten sind, seien beispielsweise genannt: Butylhydroxytoluol, Butylhydroxyanisol, Ascorbylpalmitat, Ascorbinsäure, Natrium-Ascorbat und d,l-α-Tocopherol bzw d,l-α-Tocopherolester.

Im Falle von Tocopherol-haltigen Solubilisaten konnten überraschenderweise große Mengen an Ascorbinsäure und Natrium-Ascorbat in der wäßrigen Phase gelöst werden, ohne daß es im Solubilisat zu Ausfällungen kam. Neben einer zusätzlichen Vitamin C-Anreicherung dieser flüssigen Carotinoid-Formulierung hat dies den Vorteil, daß diese wasserlöslichen Antioxidanskomponenten beim Verdünnen der Solubilisate mit sauerstoffhaltigem Wasser bzw. wäßrigem System auf Anwenderkonzentration von ca. 1 bis 100 ppm Carotinoid den gelösten Sauerstoff effizient abfangen können, so daß die Carotinoide geschützt bleiben.

Die erfindungsgemäßen Solubilisate erfüllen die Erfordernisse der mikrobiologischen Prüfung zur Keimzahlbestimmung nach DAB 10 (DAB: Deutsches Arzneibuch). Danach übertrifft die Reduktion aller Testkeime die Anforderungen des DAB an die Konservierungseigenschaften von oralen Zubereitungen. Darüberhinaus kann durch Zusatz von Lebensmittel-zugelassenen Konservierungsstoffen wie beispielsweise Sorbinsäure, Natrium-Sorbat, Benzoesäure, Natrium-Benzoat, Benzylalkohol, Butylhydroxytoluol oder PHB-Ester wie 4-Hydroxy-methylbenzoat oder 4-Hydroxy-propylbenzoat in einer Konzentration von 0 bis 200 Gew.-%, vorzugsweise 10 bis 150 Gew.-%, besonders bevorzugt 15 bis 80 Gew.-%, bezogen auf das/die Carotinoid(e), eine, bei längerer Lagerzeit nicht immer auszuschließende, unerwünschte mikrobielle Zersetzung des Solubilisats verhindert werden.

Eine weitere Möglichkeit zur Vermeidung mikrobiologischer Keimbildung bei langen Lagerzeiten ist die Erniedrigung der Wasseraktivität im Solubilisat durch Zugabe von Zucker und/oder Zuckeralkohole, wie beispielsweise Saccharose, Glucose, Fructose, Lactose, Invertzucker, Sorbit, Mannit oder Glycerin.

Die kontinuierliche Herstellung von Solubilisaten führt zu wäßrigen Dispersionen von sehr kleinen Micellen mit einem mittleren Partikeldurchmesser von 10 bis 200 nm, vorzugsweise kleiner 100 nm, wobei das/die Carotinoid(e) gelost im Micellinneren vorliegt. Überraschenderweise sind die Solubilisate trotz der hohen Wirkstoffkonzentration von bis zu 10 Gew.-% physikalisch und chemisch stabil. Eine Ausfällung der an sich wasserunlöslichen Carotinoide wurde auch bei den hohen Konzentrationen sowohl an Wirk- als auch an Zusatzstoffen nicht beobachtet.

Die Verwendung der konzentrierten Carotinoid-Solubilisate liegt überwiegend im Bereich der Lebensmittelfärbung, speziell im Einfärben von Getränken, die optisch klar bleiben sollen. Die Solubilisate sind sowohl mit Ascorbinsäure, wie bereits oben erwähnt, als auch mit anderen in der Lebensmittel- und Getränke-Industrie gebräuchlichen Säuren, wie beispielsweise Fruchtsäuren sehr gut verträglich. Weiterhin lassen sich mit diesen flüssigen Carotinoid-Formulierungen Fette, wie Butter und Margarine sowie Milchprodukte färben.

In den nachfolgenden Beispielen wird die Durchführung des erfindungsgemäßen Verfahrens näher erläutert.

### Beispiel 1

Eine auf 65°C vorgewärmte Suspension von 500 g β-Carotin in 2500 g Polyoxyethylen(20)sorbitanmonostearat (Tween® 60) wurde bei einem Durchsatz von 2,2 kg/h einer, in ein auf 190°C temperiertes Ölbad getauchten Heizschlange mit einem Innendurchmesser von 2 mm und einer Länge von 12 m zugeführt. Bei einer Temperatur von 164°C nach Austritt aus dem Wärmeaustauscher und nach einer Verweilzeit von 62 s wurde das β-Carotin im Emulgator gelöst. In einer anschließenden Mischkammer wurde die β-Carotin-Lösung mit 25°C warmen Wasser (Durchsatz: 5,4 kg/h) bei einer Mischungstemperatur von 62°C turbulent vermischt. Das Solubilisat wurde bei einem Druck von 20 bar über ein Druckbegrenzer-Ventil ausgetragen. Man erhielt eine dunkelrot gefärbte, mizellare β-Carotin-Lösung mit einem β-Carotin Gehalt von 4,4 Gew.-% und einer Mizellgröße von 20 nm.

### Beispiel 2

Eine auf 70°C vorgewärmte Suspension von 500 g β-Carotin in 2300 g Polyoxyethylen(20)sorbitanmonostearat (Tween® 60) und 207 g d,l-α-Tocopherol wurde bei einem Durchsatz von 2,2 kg/h einer, in ein auf 190°C temperiertes Ölbad getauchten Heizschlange mit einem Innendurchmesser von 2 mm und einer Länge von 12 m zugeführt. Bei einer Temperatur von 164°C nach Austritt aus dem Wärmeaustauscher und einer Verweilzeit von 62 s wurde das β-Carotin im Emulgator gelöst. In einer anschließenden Mischkammer wurde die β-Carotin-Lösung mit 25°C warmen Wasser (Durchsatz: 5,4 kg/h) bei einer Mischungstemperatur von 61°C turbulent vermischt. Das Solubilisat wurde bei einem Druck von 20 bar über ein Druckbegrenzer-Ventil ausgetragen. Man erhielt eine dunkelrot gefärbte, mizellare β-Carotin-Lösung mit einem β-Carotin Gehalt von 4,4 Gew.-% und einem Gehalt an Tocopherol von 2,0 Gew.-%. Die mittlere Mizellgröße betrug 29 nm.

### Beispiel 3

Die Solubilisierung erfolgte analog Beispiel 1. Als wäßrige Phase wurden jeweils eine 10, 20 bzw. 30 Gew.-%ige, wäßrige Glycerinlösung verwendet. Die Versuchsergebnisse sind in Tabelle 1 zusammengefaßt.

**Tabelle 1**

| | 10 Gew.-% Glycerin | 20 Gew.-% Glycerin | 30 Gew.-% Glycerin |
|---|---|---|---|
| Durchsatz Suspension [kg/h] | 2,2 | 2,2 | 2,3 |
| Durchsatz wäßrige Phase [kg/h] | 5,4 | 5,4 | 5,4 |
| Gehalt β-Carotin [Gew.-%] | 4,4 | 4,4 | 4,6 |
| Mizellgröße [nm] | 17 | 18 | 28 |

### Beispiel 4

Die Versuchsdurchführung erfolgte analog Beispiel 2. In drei unteschiedlichen Versuchen wurde der Einfluß verschiedener Mengen an d,l-α-Tocopherol auf die Solubilsateigenschaften untersucht. Die Versuchsergebnisse sind in Tabelle 2 zusammengefaßt.

**Tabelle 2**

| | 0,5 Gew.-% Tocopherol | 3,0 Gew.-% Tocopherol | 4,0 Gew.-% Tocopherol |
|---|---|---|---|
| Durchsatz Suspension [kg/h] | 2,2 | 2,4 | 2,3 |
| Durchsatz wäßrige Phase [kg/h] | 5,4 | 5,4 | 5,4 |
| Gehalt β-Carotin [Gew.-%] | 4,5 | 3,6 | 3,9 |
| Mizellgröße [nm] | 17 | 74 | 95 |

### Beispiel 5

Die Versuchsdurchführung erfolgte analog Beispiel 2. Anstelle von Wasser wurde eine Lösung von je 200 g Ascorbinsäure und 200 g Natrium-Ascorbat pro kg Wasser verwendet. Das erhaltene Solubilisat besaß folgende Zusammensetzung: 3,9 Gew.-% β-Carotin, 2,0 Gew.-% Tocopherol, 9,9 Gew.-% Natrium-Ascorbat, 9,9 Gew.-% Ascorbinsäure. Mizellgröße: 19 nm.

### Beispiel 6

Eine auf 50°C vorgewärmte Suspension von 200 g einer 20 Gew.-%igen Dispersion von Apocarotinal in Erdnußöl in 480 g Polyoxyethylen(20)sorbitanmonostearat (Tween® 60) und 16 g Tocopherol wurde bei einem Durchsatz von 1,0 kg/h einer, in ein auf 170°C temperiertes Ölbad getauchten Heizschlange mit einem Innendurchmesser von 2 mm und einer Länge von 12 m zugeführt. Bei einer Temperatur von 138°C nach Austritt aus dem Wärmeaustauscher und einer Verweilzeit von 136 s wurde das Apocarotinal im Emulgator gelöst. In einer anschließenden Mischkammer wurde die Apocarotinal-Lösung mit 25°C warmen Wasser (Durchsatz: 8,3 kg/h) bei einer Mischungstemperatur von 40°C turbulent vermischt. Das Solubilisat wurde bei einem Druck von 30 bar über ein Druckbegrenzer-Ventil ausgetragen. Man erhielt eine dunkelrot gefärbte, mizellare Apocarotinal-Lösung mit einem Apocarotinal Gehalt von 0,59 Gew.-%, einem d,l-α-Tocopherol-Gehalt von 0,25 Gew.-% und einer Mizellgröße von 21 nm.

### Beispiel 7

Eine auf 50°C vorgewärmte Suspension von 40 g Apocarotinal in 500 g Polyoxyethylen(20)sorbitanmonopalmitat (Tween® 40) und 32 g d,l-α-Tocopherol wurde bei einem Durchsatz von 1,0 kg/h einer, in ein auf 180°C temperiertes Ölbad getauchten Heizschlange mit einem Innendurchmesser von 2 mm und einer Länge von 12 m zugeführt. Bei einer Temperatur von 150°C nach Austritt aus dem Wärmeaustauscher und einer Verweilzeit von 136 s wurde das Apocarotinal im Emulgator gelöst. In einer anschließenden Mischkammer wurde die Apocarotinal-Lösung mit 25°C warmen Wasser (Durchsatz: 8,3 kg/h) bei einer Mischungstemperatur von 40°C turbulent vermischt. Das Solubilisat wurde bei einem Druck von 30 bar über ein Druckbegrenzer-Ventil ausgetragen. Man erhielt eine dunkelrot gefärbte, mizellare Apocarotinal-Lösung mit einem Apocarotinal Gehalt von 0,6 Gew.-%, einem d,l-α-Tocopherol Gehalt von 0,6 Gew.-% und einer Mizellgröße von 35 nm.

### Beispiel 8

Ein Volumenteil Solubilsat aus Beispiel 1 wurde mit demselben Volumen einer 1 Gew.-%igen, wäßrigen Lösung eines Entschäumers gemischt, unter definierten Bedingungen 30 s geschüttelt und bei definierter Geometrie die Höhe der Schaumkrone in cm in Abhängigkeit der Zeit gemessen. Als Vergleich wurde das Solubilisat mit demselben Volumen Wasser gemischt. Gemäß Tabelle 3 wurde bei Zusatz von Entschäumer deutlich weniger Schaumbildung und/oder ein schnellerer Zerfall des gebildeten Schaumes beobachtet.

**Tabelle 3**

| Entschäumer | Schaumhöhe (Start) [cm] | Schaumhöhe (5 min) [cm] | Schaumhöhe (10 min) [cm] |
|---|---|---|---|
| ohne | 2,5 | 1,5 | 1,0 |
| Sorbitanmonopalmitat | 1,5 | 1,4 | 1,3 |
| Essigsäureester von Mono-/Diglyceriden | 3,0 | 1,0 | 0,7 |
| Lecithin | 1,0 | 0,8 | 0,7 |

### Beispiel 9

In einem Becherglas wurden 100 g Fructose, 5 g Zitronensäure und 1 g Vitamin C in 800 g Wasser gelöst. In einem zweiten Becherglas wurden in eine Lösung von 0,25 g Xanthan in 100 g Wasser 15 ml Orangenessenz und 65 mg eines 4 Gew.-%igen β-Carotin-Solubilisates einemulgiert und mit einem Ultraturrax "T25" 2 Minuten bei 1000 U/min homogenisiert. Anschließend wurden die Inhalte beider Bechergläser unter Rühren vereinigt, über eine Kurzzeiterhitzungsanlage pasteurisiert und in Flaschen abgefüllt. Das Getränk wies eine klare, brillante Gelbfärbung mit einem β-Carotin-Gehalt von 2,5 ppm auf.

## Patentansprüche

1. Verwendung von Carotinoid-Solubilisaten zum Färben von Lebensmitteln und pharmazeutischen Zubereitungen, dadurch gekennzeichnet, daß man Carotinoid-Solubilisate einsetzt, die hergestellt sind, indem man eine Suspension, enthaltend 1 bis 40 Gew.-% eines oder mehrerer Carotinoide, 20 bis 90 Gew.-% eines oder mehrerer nichtionogener Emulgatoren und 0 bis 50 Gew.-% weiterer Zusatzstoffe, kurzzeitig auf Temperaturen von 120 bis 200°C erhitzt, und die homogene Lösung mit solchen Mengen Wasser oder einer wäßrigen Lösung, enthaltend hydrophile Antioxidantien sowie gegebenenfalls weitere grenzflächenaktive Zusatzstoffe, bei 10 bis 95°C turbulent vermischt, daß ein Solubilisat mit 0,5 bis 10 Gew.-% an Carotinoiden entsteht.

2. Verwendung von Carotinoid-Solubilisaten nach Anspruch 1 mit einem Carotinoid-Gehalt von 1,5 bis 6 Gew.-%.

3. Verwendung von Carotinoid-Solubilisaten nach Anspruch 1, dadurch gekennzeichnet, daß sie neben nichtionogenen Emulgatoren mit einem HLB-Wert von 12 bis 16 gegebenenfalls weitere nichtionogene Emulgatoren mit einem HLB-Wert kleiner 5 enthalten.

4. Verwendung von Carotinoid-Solubilisaten nach Anspruch 1, dadurch gekennzeichnet, daß sie zusätzlich 0 bis 40 Gew.-% Antioxidantien enthalten.

5. Verwendung von Carotinoid-Solubilisaten nach Anspruch 1, dadurch gekennzeichnet, daß sie eine Partikelgröße von 10 bis 200 nm aufweisen.

6. Verwendung von Carotinoid-Solubilisaten nach den Ansprüchen 1 bis 5 zum Färben von Getränken.

7. Verwendung von Carotinoid-Solubilisaten nach den Ansprüchen 1 bis 5 zum Färben von Margarine und Milchprodukten.
